# EUROPEAN PATENT APPLICATION

(11) **EP 4 062 890 A1**
(43) Date of publication of application: **28.09.2022**
(21) Application number: 22161676.6
(22) Date of filing: 11.03.2022
(51) Int. Cl.: A61H 9/00

(54) **INFLATABLE COMPRESSION SLEEVE**

(30) Priority: 14.03.2021 IL 28146721
(71) Applicant: Mego Afek AC Ltd., 3004200 Doar Afek (IL)
(72) Inventor: BEN-NUN, Asher, 2172111 Carmiel (IL)
(74) Representative: McNamara, Kathryn

(57) **Abstract**

An inflatable compression sleeve comprises a plurality of consecutive inflatable pressure cells and a breathing chamber associated with at least one pair of adjacent pressure cells, configured to contain air therein. The breathing chamber is configured to be in fluid communication with an exterior of the sleeve via each of inner and outer surfaces of the sleeve. When the volume of the breathing chamber is decreased from as a result of the adjacent pressure cells being brought into the inflated configuration, air from the breathing chamber is expelled to the exterior of the sleeve via the inner surface, and when the volume of the breathing chamber is increased as a result of the adjacent air cells being brought into the deflated configuration thereof, air is drawn into the breathing chamber from the exterior of the sleeve via the outer surface.

## Description

### TECHNOLOGICAL FIELD

The present disclosure relates to a compression sleeve designed for applying pressure to a body, particularly, for the purposes of compression therapy.

### BACKGROUND

Inflatable compression sleeves are used in many kinds of medical treatment, such as the treatment of swelling in a limb, for example in lymphedema. Such sleeves can be wrapped around a limb, such as an arm, leg, foot or the like and fastened using any conventional fastening mechanisms, such as straps, buckles, hook and loop, zipper, lacing or the like. The sleeve comprises a plurality of pressure cells which are successively inflated after fastening the sleeve to the limb, in order to apply pressure to the limb.

US 6,846,295 B1 discloses an inflatable compression sleeve, in which adjacent pressure cells overlap at least when inflated, in order to ensure continuity and uniformity of applied pressure over the area enclosed by the sleeve. This prevents areas of lack of or lower applied pressure at the seams or edges between pressure cells, for example. Other inflatable compression sleeves having overlapping pressure cells for application of compression therapy are exemplified by US 5,830,164 and WO 2006/048619 A1.

### GENERAL DESCRIPTION

In accordance with the presently disclosed subject matter, there is provided an inflatable compression sleeve, of the kind disclosed in US 6,846,295 incorporated herein by reference, which can be adapted to be wrapped around a limb of a patient, for use in compression therapy, e.g. for the treatment of lymphedema.

In particular, the inflatable compression sleeve according to the presently disclosed subject matter, is of the kind comprising an outer surface configured, in use, to face away from the body of a patient and an opposing inner surface configured, in use, to face towards the body of a patient, the outer and inner surfaces defining therebetween a through-thickness direction of the sleeve perpendicular to a longitudinal direction thereof; and a plurality of consecutive inflatable pressure cells arranged along the longitudinal direction such that, when viewed along the through-thickness direction, each two adjacent pressure cells at least partially overlap at least in one of an inflated and a deflated configuration thereof.

The inflatable compression sleeve according to the presently disclosed subject matter, is configured to provide improved comfort to the user especially for long-term use due to its comprising a breathing chamber associated with at least one pair of adjacent pressure cells, configured to contain air therein and to have a first volume when both adjacent pressure cells are in the deflated configuration and a second, smaller volume when both adjacent pressure cells are in the inflated configuration; the breathing chamber being configured to be in fluid communication with an exterior of the sleeve via each of the inner and outer surface of the sleeve such that, when the volume of the breathing chamber is decreased from the first volume to the second volume as a result of the adjacent pressure cells being brought into the inflated configuration thereof, air from the breathing chamber is expelled to the exterior of the sleeve via the inner surface, and when the volume of the breathing chamber is increased from the second volume to the first volume as a result of the adjacent air cells being brought into the deflated configuration thereof, air is drawn into the breathing chamber from the exterior of the sleeve via the outer surface.

The inflatable compression sleeve according to the presently disclosed subject matter can comprise a plurality of the above breathing chambers, each disposed between adjacent pressure cells of one pair of such cells.

One or more of the breathing chambers can comprise an air inlet at the outer surface of the compression sleeve via which air can be drawn into the breathing chamber and an air outlet at the inner surface of the compression sleeve via which air can be expelled from the breathing chamber. At least one of the air inlet and air outlet can comprise, or can be in the form of, a non-return valve.

One or more of breathing chambers can comprise a filler member made of a elastically compressible material and configured to hold air therein when in a normal state and expel air therefrom when brought into a compressed state. The compressible material can comprise, or is in the form of, an open-cell foam.

The inflatable compression sleeve can be of the kind comprising a first sheet made of a flexible fluid-impervious material and having an inner and an outer surface, the outer surface of the first sheet constituting the outer surface of the sleeve; a second sheet made of a flexible fluid-impervious material and sealingly fixed to the first sheet so as to form said pressure cells; and a third sheet made of a flexible material and having an inner and an outer surface, the outer surface of the third sheet constituting said inner surface of the sleeve. In such compression sleeve, each pressure cell can extend between a pair of connection lines oriented transversely to the longitudinal direction of the sleeve and defining first and second strip regions on the respective first and second sheets; a width of the second strip region between said pair of transverse connection lines, at least for the majority of the pressure cells, can be greater than that of the first strip region, to form pleats along the transverse connection lines, which are maintained in their pleated state at least in the inflated configuration of the pressure cells; adjacent transverse connection lines of adjacent pressure cells can be spaced from each other in the longitudinal direction of the sleeve; and the pressure cells, at least in the inflated configuration, can have said second strip region of one pressure cell overlapping the second strip region of the adjacent pressure cell. The third sheet can be configured to maintain the second strip regions in their pleated state in both the inflated and deflated configurations of the pressure cells.

The width of material of the second strip region can be greater than the width of material of the first strip region by about 50% of the width of the first strip region. In the deflated configuration, each pleat can overlap the second strip region of the adjacent cell by about 25% to 35% of the width thereof.

The compression sleeve has a proximal end and a distal end and the pleats can be oriented in the direction towards the proximal end thereof.

Each pressure cell can be in fluid communication with the outer surface of the sleeve and can have a fluid opening to enable at least one of direct inflation or direct deflation of the pressure cell.

Reverting to the breathing chambers between adjacent pressure cells, each such chamber can extend between the first and third sheets and can have a width along the first sheet in the longitudinal direction corresponding to a distance between the adjacent connection lines of the adjacent pressure cells. The width of the breathing chamber along the first sheet in the longitudinal direction can be essentially smaller than that along the third sheet.

When the breathing chamber comprises the filler member, such member can be configured to be compressed between the second strip regions of the adjacent pressure cells when the pressure cells are brought into the inflated configuration.

The third sheet can be made of an air-permeable material and thus constitute the air outlet of the breathing chamber at the inner surface of the compression sleeve via which air can be expelled from the breathing chamber.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1** shows a schematic plan view of a flattened inflatable compression sleeve of the kind to which the presently disclosed subject matter refers, with pressure cells in a deflated configuration;
**Fig. 2A** shows a schematic side view of an inflatable compression sleeve, in accordance with one example of the presently disclosed subject matter, with pressure cells in an inflated configuration;
**Fig. 2B** shows a schematic side view of the inflatable compression sleeve of Fig. 2A, with the pressure cells in a deflated configuration;
**Fig. 3** shows a schematic side view of an inflatable compression sleeve, in accordance with another example of the presently disclosed subject matter, with pressure cells in an inflated configuration;
**Fig. 4** shows a schematic cross-sectional side view of an inflatable compression sleeve, of the kind to which the presently disclosed subject matter refers, wrapped around a leg and foot;
**Fig. 5** shows a schematic side view of an inflatable compression sleeve, according to a further example of the presently disclosed subject matter, with the pressure cells in a deflated configuration; and
**Fig. 6** shows a schematic side view of the inflatable compression sleeve of Fig. 5, with the pressure cells in an inflated configuration.

### DETAILED DESCRIPTION OF EMBODIMENTS

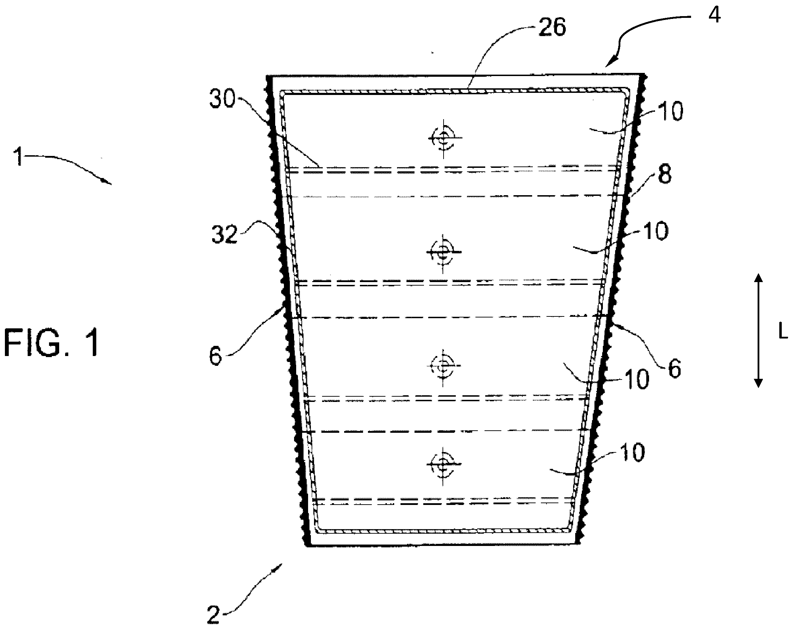

Fig. 1 shows generally a compression sleeve 1 of the kind to which the present disclosure refers, in a flat state as manufactured and/or as deflated. The sleeve is adapted to be wrapped around a human body or a limb thereof, e.g. a leg of a patient, to apply pressure thereon for the purposes of compression therapy, in particular to alleviate swelling therein.

As shown in Fig. 1, the sleeve 1 can have a generally trapezoidal shape, i.e. having two parallel edges being distal and proximal ends 2 and 4, respectively, and sides 6. The distal and proximal ends 2, 4 define a longitudinal direction L therebetween. Although shown as trapezoidal in shape, with the sides 6 diverging from the distal to the proximal end, the sleeve 1 can have any other shape in accordance with a limb or part of a patient's body to be treated. For the fixation of the sleeve 1 in its operative position, i.e. when wrapped around a limb, the sleeve 1 can be provided with fasteners which are shown in FIG. 1 as a zipper 8, but which can alternatively be in the form of hook and loop fasteners, laces or of any other conventional design.

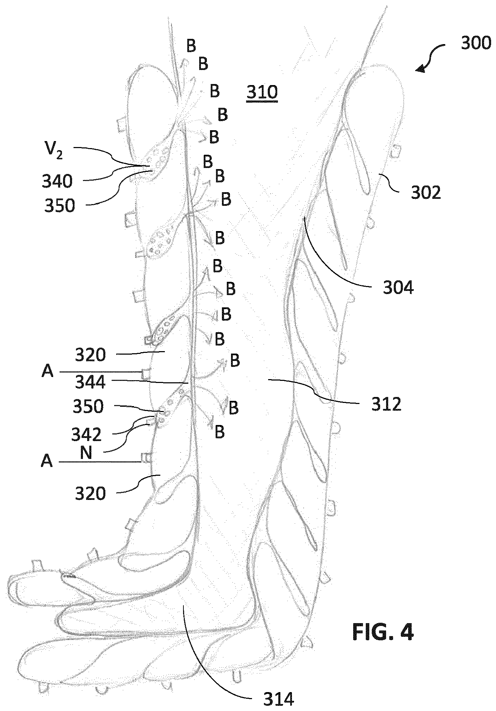

The sleeve 1 has a plurality of pressure cells 10 having fluid openings 12 configured for being connected to a fluid line (not shown) for the inflation and deflation of the cells 10 by a fluid 14 (not shown) to apply pressure on a limb of a patient, such as a leg of a patient as shown in FIG. 4, in a controlled manner. The fluid may be in a liquid or gaseous state. In particular, it may be in the form of air, in which case the fluid openings are in the form of air inlets. The fluid openings are configured to be connected by means of hoses, tubes, conduits and the like (not shown) to an inflating device such as a compressor with distributor valve (not shown) so as to allow the inflation of the pressure cells 120 in a controlled manner. The air inlet A can also function as an air outlet when the pressure cells 120 are being deflated.

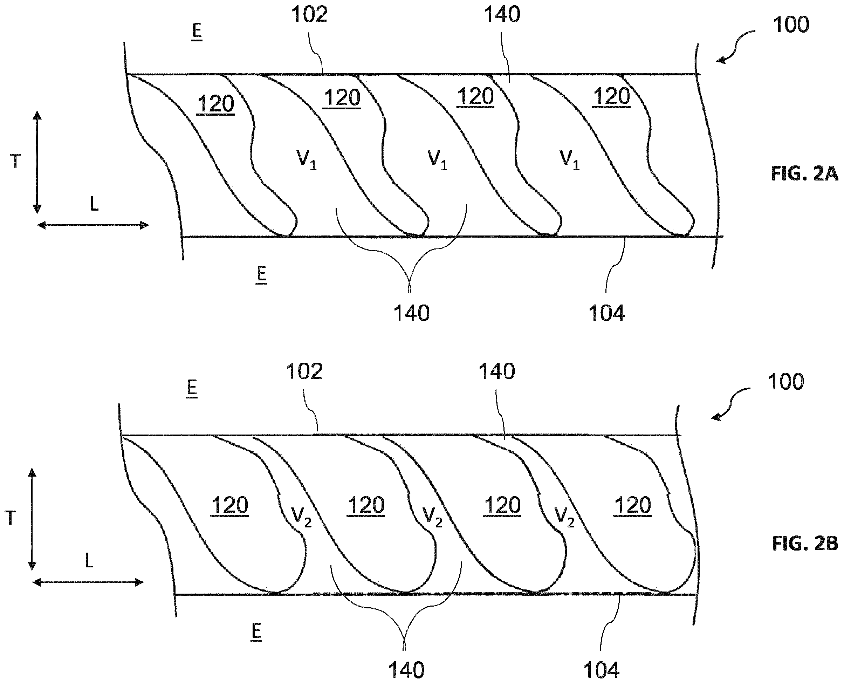

Compression sleeves described below with reference to Figs. 2A to 6 should be considered as having all features described of the compression sleeve 100 described above.

As seen in Figs. 2A and 2B, and as described in more detail below in both deflated and inflated configurations, the pressure cells 120 overlap their immediately adjacent neighboring pressure cells. In the presently disclosed examples, the overlapping is in the direction towards the proximal end 4 of the sleeve, i.e. the pressure cells in both states are oriented so that, when the sleeve is in use, their portions located closer to the body of a patient are disposed closer to the proximal end of the sleeve than their portions located further from the patient's body.

Figs. 2A and 2B show an inflatable compression sleeve 100 according to one example of the presently disclosed subject matter, having a longitudinal direction L. The compression sleeve 100 comprises an outer surface 102 configured, in use, to face away from the body of a patient and an opposing inner surface 104 configured, in use, to face towards the body of a patient. The outer and inner surfaces 102, 104 define therebetween a through-thickness direction T of the compression sleeve 100 perpendicular to the longitudinal direction L.

The compression sleeve 100 comprises a plurality of consecutive inflatable pressure cells 120 arranged along the longitudinal direction L. Whilst not shown in Figs. 2A and 2B, each pressure cell 120 is provided with an air inlet, e.g. in the form of a nipple, via which the pressure cell can be inflated and deflated (see Figs. 1 and 4 to 6).

In general, when viewed along the through-thickness direction, each two adjacent pressure cells 120 at least partially overlap in at least in one of an inflated and a deflated configuration of the pressure cells 120. In the particular example of Figures 2A and 2B, which shows the pressure cells in both configuration, it can be seen that when viewed along the through-thickness direction, each two adjacent pressure cells 120 at least partially overlap at least in the inflated configuration of the pressure cells 120.

The compression sleeve 100 of the present example further comprises a breathing chamber 140 between each two adjacent pressure cells 120, configured to contain air therein and to have a first volume V₁ when both adjacent pressure cells 120 are in the deflated configuration and a second, smaller volume V₂ when both adjacent pressure cells 120 are in the inflated configuration. Alternatively, such breathing chambers can be formed only between selected pairs of adjacent pressure cells rather than each pair of adjacent pressure cells.

The breathing chamber 140 is configured to be in fluid communication with an exterior E of the compression sleeve 100 via each of the inner and outer surface 104, 102 such that, when the volume of the breathing chamber 140 is reduced from the first volume V₁ to the second volume V₂, air from the breathing chamber 140 is expelled to the exterior of the sleeve via the inner surface 104, and when the volume of the breathing chamber 140 is increased from the second volume V₂ to the first volume V₁, air is drawn into the breathing chamber 140 from the exterior of the sleeve 100 via the outer surface 102.

In general, breathing chambers of a compression sleeve of the presently disclosed subject matter, can have such as structure as to ensure that air can flow into its breathing chambers only via the outer surface of the sleeve and out towards the body of a wearer of the compression sleeve only via the inner surface of the sleeve. In this manner, fresh air flow can be provided to the body of the wearer, and drawing in of sweat or other undesirable or contaminated matter from the body of a wearer into the compression sleeve can be prevented. To this end, the breathing chambers can have an air inlet at the outer surface and an air outlet at the inner surface of the sleeve, and for example at least one of them can be in the form or comprise a non-return valve.

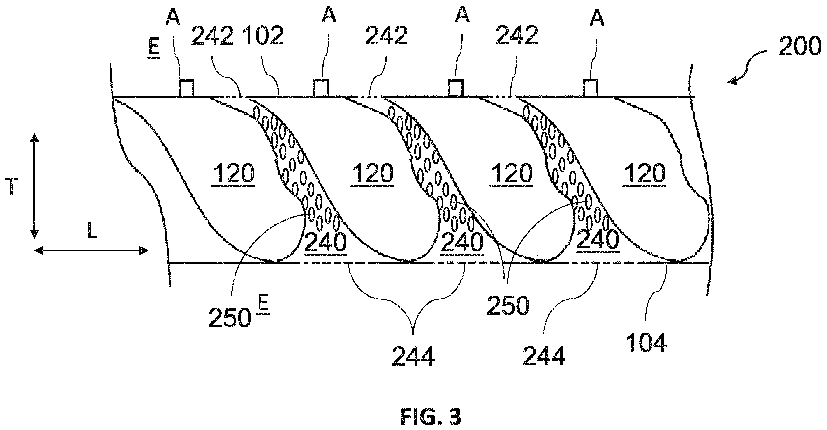

Fig. 3 shows a compression sleeve 200 similar to that of Figs. 2A and 2B, with like features denoted by like reference signs.

Whilst shown only with respect to the example of Fig. 3, in both the compression sleeve 200 of Fig. 3 and the compression sleeve 100 of Figs. 2A and 2B, each pressure cell 120 is provided with an air inlet or nipple A, which can be provided with a collar in the pressure cell 120. The air inlet A is configured to be connected by means of hoses, tubes, conduits and the like (not shown) to an inflating device such as a compressor with distributor valve (not shown). In this manner, the inflation of the pressure cells 120 can be achieved in a controlled manner. The air inlet A can also function as an air outlet when the pressure cells 120 are being deflated.

The breathing chamber 240 of the compression sleeve 200 is identical to the breathing chamber 140 of the compression sleeve 100, except that the breathing chamber 240 comprises an air inlet 242 at the outer surface 102 of the compression sleeve 200, via which air can be drawn into the breathing chamber 240 and an air outlet 244 at the inner surface 104 of the compression sleeve 200, via which air can be expelled from the breathing chamber 240.

At least one of the air inlet 242 and air outlet 244 can comprise, or be in the form of, a non-return valve. This ensures that in use, air flows into the compression sleeve 200 via the air inlet 242 only and out towards the body of a wearer of the compression sleeve 200 via the air outlet 244 only.

In general, in any compression sleeve of the presently disclosed subject matter, the breathing chamber can either be free of a filler or can comprises a filler member made of an elastically compressible material configured to hold air therein when in a normal state, i.e. a state at rest in which the material is in a relatively relaxed state and expel air therefrom when brought into a relatively compressed state. One example of such materials is an open-cell foam.

As shown in the specific example of Fig. 3, the breathing chambers comprise an open-cell foam filler material/member 250, which is in a compressed state in Fig. 3 since the pressure cells 120 are in the inflated configuration.

Fig. 4 shows an inflatable compression sleeve 300 wrapped around the body of a patient, in this case a limb 310, comprising a leg portion 312 and a foot portion 314. The compression sleeve 300 has an outer surface 302 facing away from the limb 310 and an inner surface 304 facing towards the limb. As can be seen, the inflatable compression sleeve comprises a plurality of overlapping pressure cells 320, which are shown in the inflated configuration. At least on the part of the inflatable compression sleeve 300 surrounding the leg portion 312, between each two consecutive pressure cells 320 is a breathing chamber 340. In this example, the breathing chambers 340 comprise a filler member 350 made of an open-cell foam filler material and disposed at the front of the sleeve.

On the outer surface 302, each of the breathing chambers 340 is provided with a an air inlet 342 provided with a non-return valve N and on the inner surface 304, each of the breathing chambers 340 is provided with an air outlet 344. Each air inlet is provided or is in the form of a non-return valve N configured to allow air to pass only from the exterior E of the compression sleeve into the breathing chambers 340, but not in the opposite direction through the air inlet 342.

In Fig. 4 the air inlets and outlets of the breathing chambers 340 happen to be aligned with the front of the leg when the compression sleeve is wrapped around the leg, but in fact these outlets could instead be aligned with any part of the leg depending on how the compression sleeve is wrapped around the leg.

As the pressure cells 320 of the compression sleeve 300 are brought from the deflated configuration to the inflated configuration, the volume of the breathing chambers 340 is reduced, from a larger volume (not shown) to the smaller volume shown in Fig. 3, and the filler member 350 is compressed. Due to the presence of the non-return valves N, some of the air in the breathing chambers 340 is forced out of the air outlets 344 towards the body of the patient, which in this example is at least the leg portion 312, as shown by diverging arrows B.

As the pressure cells 320 of the compression sleeve 300 are brought from the inflated configuration to the deflated configuration, the volume of the breathing chambers 340 is increased, from the smaller volume V₂ shown in Fig. 3 to a larger volume (not shown). The expansion of the breathing chambers 340 can be assisted by elastic potential energy stored in the compressed filler material of the filler member 350. As the breathing chambers 340 expand, fresh air is drawn into the breathing chambers 340 via the air inlets.

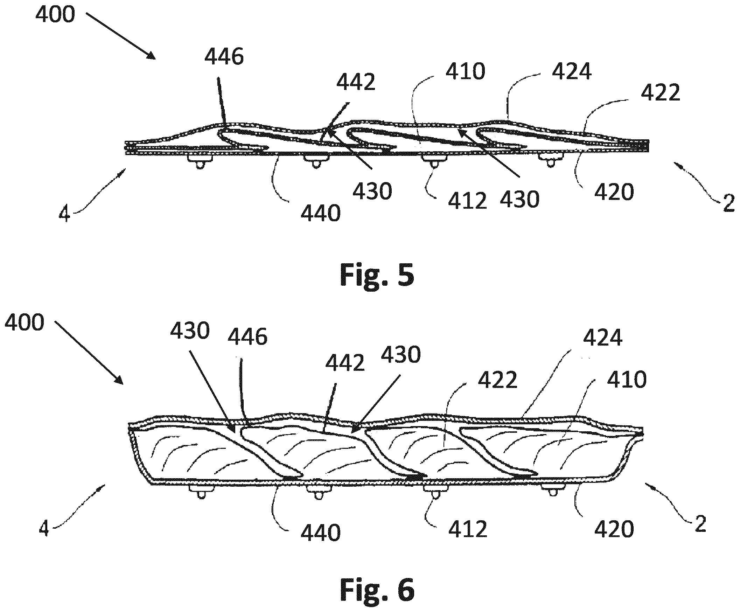

One example of the general construction which an inflatable compression sleeve according to the presently disclosed subject matter, and particularly the sleeves 100, 200 and 300 described above, can have will now be further described with reference to an exemplary sleeve 400 shown in Figs. 5 and 6 and having all features shown and described above.

The sleeve 400 is made of first, second and third sheets 420, 422 and 424 of a flexible material, which constitute the sleeve's respective outer, intermediate and inner surfaces or layers and which are all connected by a peripheral connection line 26 shown in FIG. 1, circumferentially extending along the distal and proximal ends 2 and 4 and sides 6 of the sleeve.

The first and second sheets 420 and 422 are made of a fluid-impervious material, such as for example, nylon coated with polyurethane, and they are sealingly connected with one another by a plurality of transverse connection lines 30 oriented transversely to the longitudinal direction of the sleeve as shown in Fig. 1, and defining therebetween the pressure cells 10, 410. The sheets 420 and 422 are further sealingly connected with each other by lateral connection lines 32 shown in Fig. 1, directed along the sides 6 of the sleeve, whereby it is ensured that each cell 10, 410 has fluid-tight boundaries. The sealing connection between the sheets 420 and 422 along the transverse and lateral connection lines 30 and 32 may be provided by such means as welding, adhesive bonding or radio frequency treatment, and the like. The transverse connection lines 30 that are located adjacent the distal and proximal ends 2 and 4 of the sleeve, and the lateral connection lines 32 that are located adjacent the sides 6 of the sleeve coincide, in the sleeve 1, with the peripheral connection lines 26 seen in Fig. 1, and are made simultaneously therewith. However, this does not necessarily need to be the case.

As best seen in Figs. 5 and 6, the transverse connection lines 30 divide the sheets 420 and 422 into pairs of respective first and second strip regions 440 and 442, which define therebetween the pressure cells 410. The breathing chambers 430 are defined between the pressure cells 410. The fluid openings 412, which are the air inlets for the pressure cells 410, are formed in the first strip regions 440. The second strip regions 442, in all the pressure cells except for the most proximal cell that is disposed adjacent the proximal end 4 of the sleeve, are wider than the first strip regions 440, i.e. the width of the material of which the second strip regions 442 are made is greater than that of the first strip regions 440, preferably by an amount of about 50%. Due to this difference, the second strip regions 442 form pleats 446 which extend along the transverse connection lines 30 and are oriented in the direction towards the proximal end 4 of the sleeve. The pleats 446 are formed so as to overlap the second strip regions 442 of immediately adjacent neighboring cells 410, both when the cells 410 are deflated (Figs. 2B and 5) and inflated (Figs. 2A and 6). In the deflated state, the extent of overlap is preferably about 25% to 35%. To fix the strip regions 442 in the pleated state, the lateral connection lines 32 pass through the pleats (not shown). It should be mentioned that in different pressure cells, the widths of the first and second strip regions, as well as the extent of overlap, might be different.

In this example, the third sheet 424 maintains the second strip regions 442 in their pleated state in both the inflated and deflated configurations of the pressure cells 410.

As shown in Figs. 5 and 6, each breathing chamber 430 between adjacent pressure cells 410 extends between the first and third sheets 420, 424 and has a width along the first sheet in the longitudinal direction L corresponding to a distance between the adjacent transverse connection lines 30 of the adjacent pressure cells 410.

In the present example, the width of the breathing chamber 430 along the first sheet 420 in the longitudinal direction L is essentially smaller than the width of the breathing chamber 430 along the third sheet in the longitudinal direction L.

In any one or more of the above examples where the breathing chamber comprises a filler material, or filler member, the filler member can be configured to be compressed between the second strip regions of the adjacent pressure cells when the pressure cells are brought into the inflated configuration.

In any one or more of the above examples, the third sheet can be made of an air-permeable material and can constitutes the air outlet of the breathing chambers at the inner surface of the compression sleeve via which air can be expelled from the breathing chambers towards the limb of the patient.

Optionally, an inflatable compression sleeve can comprise two sleeve portions, each of which acts as an individual sleeve having pressure cells designed and, manufactured in the same manner as in of any one or more of the above exemplary compression sleeves. The sleeve portions can be connected with each other by a non-pressure web, i.e. a piece of material connecting the two sleeve portions which does not provide compression. The non-pressure web may be made either as a separate piece sewn or otherwise fixed to the sleeve portions at their associated ends, or as a web cut out in the sleeve portions' common outer sheet. In the latter case, the sleeve portions each have their individual intermediate and inner sheets attached to their corresponding areas of the common outer sheet disposed on each side of the web.

While in the above examples, the drawings show the outlets and the inlets of the breathing chambers to be are aligned along the longitudinal direction of the sleeve this does not need to be the case, and the instead the breathing chamber inlets and outlets need not be linearly aligned in the longitudinal direction.

The compression sleeve of any one or more of the above examples can be reusable or disposable.

It should be understood that the above-described embodiments are only examples of a compression sleeve and method of its manufacturing according to the present invention, and that the scope of the present invention fully encompasses other embodiments which may become obvious to those skilled in the art.

## Claims

1. An inflatable compression sleeve having a longitudinal direction, comprising:
an outer surface configured, in use, to face away from the body of a patient and an opposing inner surface configured, in use, to face towards the body of a patient, the outer and inner surfaces defining therebetween a through-thickness direction of the sleeve perpendicular to the longitudinal direction thereof; and
a plurality of consecutive inflatable pressure cells arranged along the longitudinal direction such that, when viewed along the through-thickness direction, each two adjacent pressure cells at least partially overlap at least in one of an inflated and a deflated configuration thereof;
a breathing chamber associated with at least one pair of adjacent pressure cells, optionally with each of a plurality of pairs of adjacent pressure cells, configured to contain air therein and to have a first volume when both adjacent pressure cells are in the deflated configuration and a second, smaller volume when both adjacent pressure cells are in the inflated configuration; and
the breathing chamber configured to be in fluid communication with an exterior of the sleeve via each of the inner and outer surface of the sleeve such that, when the volume of the breathing chamber is decreased from the first volume to the second volume as a result of the adjacent pressure cells being brought into the inflated configuration thereof, air from the breathing chamber is expelled to the exterior of the sleeve via the inner surface, and when the volume of the breathing chamber is increased from the second volume to the first volume as a result of the adjacent air cells being brought into the deflated configuration thereof, air is drawn into the breathing chamber from the exterior of the sleeve via the outer surface.

2. A compression sleeve according to Claim 1, wherein the breathing chamber comprises an air inlet at the outer surface of the compression sleeve via which air can be drawn into the breathing chamber and an air outlet at the inner surface of the compression sleeve via which air can be expelled from the breathing chamber and, optionally, wherein at least one of the air inlet and air outlet comprises, or is in the form of, a non-return valve.

3. An inflatable compression sleeve according to Claim 1 or 2, wherein the breathing chamber comprises a filler member made of a elastically compressible material and configured to hold air therein when in a normal state and expel air therefrom when brought into a compressed state and, optionally, wherein the compressible material comprises, or is in the form of, an open-cell foam.

4. An inflatable compression sleeve according to any one of the preceding claims, comprising:
a first sheet made of a flexible fluid-impervious material and having an inner and an outer surface, the outer surface of the first sheet constituting the outer surface of the sleeve;
a second sheet made of a flexible fluid-impervious material and sealingly fixed to the first sheet so as to form said pressure cells; and
a third sheet made of a flexible material and having an inner and an outer surface, the outer surface of the third sheet constituting said inner surface of the sleeve.

5. An inflatable compression sleeve according to Claim 4, wherein
each pressure cell extends between a pair of connection lines oriented transversely to the longitudinal direction of the sleeve and defining first and second strip regions on the respective first and second sheets;
a width of the second strip region between said pair of transverse connection lines, at least for the majority of the pressure cells, is greater than that of the first strip region, to form pleats along the transverse connection lines, which are maintained in their pleated state at least in the inflated configuration of the pressure cells;
adjacent transverse connection lines of adjacent pressure cells are spaced from each other in the longitudinal direction of the sleeve; and
the pressure cells, at least in the inflated configuration, have said second strip region of one pressure cell overlapping the second strip region of the adjacent pressure cell.

6. An inflatable compression sleeve according to Claim 5, having any one or more of the following features:
- the width of material of the second strip region is greater than the width of material of the first strip region by about 50% of the width of the first strip region; and or
- in the deflated configuration, each pleat overlaps the second strip region of the adjacent cell by about 25% to 35% of the width thereof; and/or
- the sleeve has a proximal and a distal end and the pleats are oriented in the direction towards the proximal end thereof; and/or
- the third sheet maintains the second strip regions in their pleated state in both the inflated and deflated configurations of the pressure cells.

7. An inflatable compression sleeve according any one of the preceding claims, wherein each pressure cell is in fluid communication with the outer surface of the sleeve.

8. An inflatable compression sleeve according to Claim 7, wherein each pressure cell has a fluid opening to enable at least one of direct inflation or direct deflation of the pressure cell.

9. An inflatable compression sleeve according to any one of Claims 5 to 8, wherein each breathing chamber between adjacent pressure cells extends between the first and third sheets and has a width along the first sheet in the longitudinal direction corresponding to a distance between the adjacent connection lines of the adjacent pressure cells.

10. An inflatable compression sleeve according to Claim 9, wherein the width of the breathing chamber along the first sheet in the longitudinal direction is essentially smaller than that along the third sheet.

11. An inflatable compression sleeve according to Claim 9 or 10, when dependent directly or indirectly on Claim 3, wherein the filler member is configured to be compressed between the second strip regions of the adjacent pressure cells when the pressure cells are brought into the inflated configuration.

12. An inflatable compression sleeve according to any one of Claims 4 to 11 when dependent directly or indirectly on Claim 2, wherein the third sheet is made of an air-permeable material and thus constitutes the air outlet of the breathing chamber at the inner surface of the compression sleeve via which air can be expelled from the breathing chamber.

13. An inflatable compression sleeve according to any one of the preceding claims, adapted to be wrapped around a limb of a patient.

14. An inflatable compression sleeve according to any one of the preceding claims, for use in compression therapy.

15. An inflatable compression sleeve according to any one of the preceding claims, for the treatment of lymphedema.
